Europäisches Patentamt

European Patent Office

Office européen des brevets

(19)

(11) Veröffentlichungsnummer: **0 087 708**
B1

(12) ## EUROPÄISCHE PATENTSCHRIFT

(45) Veröffentlichungstag der Patentschrift:
09.04.86

(21) Anmeldenummer: 83101628.2

(22) Anmeldetag: 21.02.83

(51) Int. Cl.⁴: **C 07 F 5/02, A 61 L 15/06,
C 09 J 3/00**

(54) Neue Boralkylverbindungen, Verfahren zu ihrer Herstellung und ihre Verwendung.

(30) Priorität: 01.03.82 DE 3207264

(43) Veröffentlichungstag der Anmeldung:
07.09.83 Patentblatt 83/36

(45) Bekanntmachung des Hinweises auf die Patenterteilung:
09.04.86 Patentblatt 86/15

(84) Benannte Vertragsstaaten:
AT BE CH DE FR GB IT LI LU NL SE

(56) Entgegenhaltungen:
US - A - 3 078 311
US - A - 3 078 313

JOURNAL OF THE AMERICAN CHEMICAL SOCIETY,
Band 81, Nr. 21, 12. November 1959, Seite 6428, H.C.
BROWN et al.: "Hydroboration"

(73) Patentinhaber: Henkel Kommanditgesellschaft auf
Aktien, Postfach 1100 Henkelstrasse 67,
D-4000 Düsseldorf-Holthausen (DE)

(72) Erfinder: Ritter, Wolfgang, Dr., Hochdahler Strasse 32,
D-4010 Hilden (DE)

## Beschreibung

Boralkylverbindungen sind bekanntlich befähigt, unter Zutritt beziehungsweise Zugabe von Sauerstoff oder Sauerstoff liefernden Verbindungen radikalische Polymerisationen bei Raumtemperatur auszulösen. Der zur Polymerisation notwendige Sauerstoff ist praktisch allgegenwärtig und braucht nicht getrennt zugegeben zu werden. Insbesondere einfache Trialkylborverbindungen wie Triethylbor oder Tri-n-Butylbor sind in diesem Zusammenhang vorgeschlagen worden. Die Verwendung von Trialkylborverbindungen als Polymerisationsinitiator wird beispielsweise in den US-PSen 34 76 727, 36 33 490 und 29 85 633 sowie der GB-PS 11 13 722 beschrieben. Die US-PS 41 67 616 beschreibt Umsetzungsprodukte von Butadien und Diboran und ihre Verwendung als Polymerisationsauslöser.

Entsprechende Boralkylverbindungen eignen sich auch als Reaktionsauslöser beziehungsweise Härter für Reaktivklebstoffe, die Systeme auf Basis polymerisierbarer Monomerer enthalten. Bekannt sind hier insbesondere die Methacrylatklebstoffe. Diese Klebstoffe enthalten neben Acrylsäure- oder Methacrylsäure-Estern als wesentlichen Bestandteil Trialkylborverbindungen, beispielsweise Triethylbor oder Tri-n-butylbor (siehe hierzu beispielsweise JA-PS 4 214 318). Solche Trialkylborverbindungen weisen aber den Nachteil auf, leicht entzündlich zu sein, so daß die Handhabung dieser Klebstoffe erhebliche Schwierigkeiten bereitet.

Man hat versucht, diesen Nachteil dadurch zu beseitigen, daß man die Trialkylborverbindungen mit 0,3 bis 0,9 Mol Sauerstoff umsetzt - siehe beispielsweise DE-OS 23 21 215 - beziehungsweise man hat versucht, die Trialkylborverbindungen mit Aminen umzusetzen, um auf diese Weise ihre Selbstentzündlichkeit herabzusetzen, vergleiche JA-PS 4 529 195.

Durch diese Maßnahmen wird die Zündtemperatur zwar in den Bereich von etwa 0 bis 70 °C verschoben, gleichwohl bleibt eine beträchtliche Unsicherheit bei der Handhabung derartiger Mischungen bestehen. Außerdem ist die Reaktivität dieser Boralkylderivate stark vermindert.

Freie Boralkyle werden bei unbegrenztem Sauerstoffzutritt bekanntlich zu Borsäureestern oxidiert, die ebenfalls nicht mehr polymerisationsauslösend wirken. Zur Sicherung der Aktivität von Boralkylverbindungen als Initiatoren wird bisher der völlige Sauerstoffausschluß bei Anwendung und insbesondere bei ihrer Dosierung - ebenso wie selbstverständlich auch bei ihrer Herstellung - als erforderlich angesehen. Dementsprechend muß die jeweils benötigte Substanzmenge unter Inertgas in völlig dichte Gefäße abgepackt und ein Sauerstoffzutritt im Vorratsgefäß ausgeschlossen werden. Die portionierten Boralkyle sind quantitativ zu verbrauchen. Wegen dieser Erschwerungen eignen sich die bisher beschriebenen Systeme für zahlreiche Anwendungszwecke nicht, beispielsweise sind sie für konstruktive Verklebungen ungeeignet.

Boralkylinitiatoren zeigen an sich gegenüber konventionellen Initiatoren für radikalische Polymerisationen wie Peroxide, Hydroperoxide oder Azoverbindungen beträchtliche Vorteile. Die mit Boralkylen ausgelösten Polymerisationen laufen auch bei niedriger Temperatur ab. Das Startersystem/Härtersystem liegt in einkomponentiger Form vor. Die Polymerisationsgeschwindigkeit kann durch Variation des Sauerstoffangebots beeinflußt werden.

Die geschilderten Nachteile der einfachen Trialkylborverbindungen und insbesondere ihre leichte Selbstentzündlichkeit können dadurch wenigstens teilweise aufgefangen werden, daß stabilere Boralkylverbindungen ausgewählt werden. Ein Beispiel hierfür ist das 9-Borabicyclo [3.3.1.]-nonan (9-BBN). Dieses Dialkylborhydrid und andere stabile Boralkyle sind jedoch in vielen Monomereninsbesondere in Estergruppen enthaltenden Monomersystemen - nur sehr schwer oder sehr langsam löslich. Hier liegt für die Praxis eine wichtige Einschränkung dieser bisher bekannten, gegen Zutritt von Luftsauerstoff stabileren Boralkylverbindungen vor.

Bekannt sind aus der Literatur (US-PSen 3078311 und 3078313 sowie J. Am. Chem. Soc. 81 (1959), 6428 und J. Am. Chem. Soc. 92 (1970), 2468) Hydroborierungsprodukte von Ethyloleat und Methyl-10-undecenoat ohne daß hier etwas zu ihrer Stabilität oder ihrer Brauchbarkeit als Polymerisationsinitiatoren gesagt ist.

Die vorliegende Erfindung geht von der Aufgabe aus, Bor;alkylverbindungen zu schaffen, welche die zuvor genannten Vorteile von Boralkylinitiatoren aufweisen und insbesondere bei der Zugabe zu Monomeren oder Monomere enthaltenden Systemen durch Oxidation mit Luftsauerstoff leicht steuerbare Polymerisationen auslösen, dabei aber in reiner Form nicht selbstentzündlich sind. Die zu entwickelnden Boralkylinitiatoren sollen weiterhin in Monomersystemen insbesondere in estergruppenhaltigen Monomeren schnell und gut löslich sein und damit besonders für die Anwendung auf dem Gebiet der Polymerisation beziehungsweise Härtung von (Meth)acrylsäureestersystemen Verwendung finden können.

Gegenstand der Erfindung ist dementsprechend in einer ersten Ausführungsform ein Verfahren zum Härten von Reaktivklebstoffen, welche auf Basis von polymerisierbaren Monomeren aufgebaut sind, durch radikalische Polymerisation mit Boralkylverbindungen als Initiatoren, das dadurch gekennzeichnet ist, daß als Boralkylverbindungen Hydrobierungsprodukte eingesetzt werden aus

a) Borwasserstoff und/oder wenigstens eine B-H-Bindung aufweisenden Organoborverbindungen, deren Kohlenwasserstoffreste jeweils bis zu 15 C-Atome aufweisen und

b) Estern oder Estergemischen von Fettsäuren und/oder Fettalkoholen mit jeweils 8 - 32 C-Atomen, die wenigstens in einem Anteil ihrer Fettsäure- und/oder Fettalkoholreste wenigstens eine additionsbereite C=C-Doppelbindung bei einer Ausgangsjodzahl der Ester von 1 - 205 aufweisen, und ihrerseits Umsetzungsprodukte solcher Fettsäuren mit den Fettalkoholen oder Ester solcher Fettsäuren mit mehrwertigen Alkoholen bzw. Ester solcher Fettalkohole mit mehrwertigen Carbonsäuren sind, wobei die mehrwertigen Alkohole bzw. mehrwertigen Carbonsäuren jeweils 2 - 10 C-Atome bei einer Funktionalität bis 6 aufweisen und wobei weiterhin

c) die im Ausgangsester vorliegenden C=C-Doppelbindungen zu wenigstens 50 % der Hydroborierung unterworfen worden sind.

In weiterer Ausführungsformen betrifft die Erfindung bestimmte neue Boralkylverbindungen und ihre Verwendung zur Auslösung von Polymerisationen in Reaktivklebstoffsystemen, die auch auf dem medizinischen bzw. dentalmedizinischen Gebiet geeignet sein können.

Diese Boralkylverbindungen mit verbesserter Stabilität gegen Zutritt von Luftsauerstoff und guter Löslichkeit in Reaktivsystemen auf Basis polymerisierbare Doppelbindungen enthaltender Monomere, sind hergestellt worden durch Umsetzung von

a) Borwasserstoff und/oder wenigstens eine B-H-Bindung aufweisenden Organoborverbindungen mit jeweils bis zu 15 C-Atomen in den Kohlenwasserstoffresten und

b) Estern oder Estergemischen von Fettsäuren und/oder Fettalkoholen mit jeweils 8 - 32 C-Atomen, die wenigstens in einem Anteil ihrer Fettsäure- oder Fettalkoholreste wenigstens eine additionsbereite C=C-Doppelbindung bei einer Ausgangsjodzahl der Ester von 1 - 205 aufweisen,

wobei sie als Reaktionskomponente b) Ester von Fettsäuren mit mehrwertigen Alkoholen bzw. Ester von Fettalkoholen mit mehrwertigen Carbonsäuren enthalten, wobei diese mehrwertigen Komponenten bei einer Funktionalität bis 6, vorzugsweise von 2 - 4, jeweils 2 - 10 C-Atome, vorzugsweise 2 - 6 C-Atome aufweisen und wobei die in den Estern ursprünglich vorliegende(n) C=C-Doppelbindung(en) zu wenigstens 50 %, vorzugsweise zu wenigstens 70 %, hydroboriert sind.

Die erfindungsgemäß eingesetzten Boralkylverbindungen eigenen sich insbesondere zur Härtung von Monomerklebstoffen beispielsweise auf Methacrylatbasis. Gegenüber herkömmlichen Boralkylhärtern besitzen sie wesentliche Vorteile, die sich unter anderem wie folgt zeigen: Die erfindungsgemäßen Boralkylverbindungen sind unter Normalbedingungen nicht selbstentzündlich und stellen vergleichsweise geringe Anforderungen bei ihrer Lagerung. Die zur Härtung der Monomerkomponenten benötigte Härtermenge ist äußerst gering. Ausgewählte Härter der erfindungsgemäßen Definition sind selbst nach längerer Lagerung an der Luft voll aktiv. Die gute Verträglichkeit von Monomersystem und Härterkomponente kann durch geeignete Wahl der als Trägermatrix dienenden Esterkomponente sichergestellt werden.

Zur Beschaffenheit und Herstellung der im Rahmen der Erfindung beschriebenen und eingesetzten Boralkylverbindungen gelten im einzelnen die folgenden Angaben:

Als Matrix dienende Ester beziehungsweise Estergemische enthalten über B-C-Bindungen als Substituenten Borwasserstoff- und/oder Organoborreste. Sofern diese borhaltigen Reste nicht den Boranrest-BH2 selber darstellen, sind diese borhaltigen Substituenten in einer weiterhin bevorzugten Ausführungsform ihrerseits am Bor mit wenigstens einer weiteren B-C-Bindung an einen oder mehrere Kohlenwasserstoffreste gebunden. Geeignete Substituenten am Bor sind damit Alkyl-, Cycloalkyl- und/oder Arylreste, die in einer oder in den beiden nicht durch die Estermatrix besetzten Valenzen des Bors vorliegen können. Liegen solche von Wasserstoff abweichenden organischen Reste in beiden Borvalenzen vor, so können sie ihrerseits zu einem Ringsystem geschlossen sein.

Diese als Polymerisationsinitiatoren verwendbaren Borverbindungen können in einfacher Weise dadurch erhalten werden, daß man das olefinische Doppelbindungen enthaltende Ester-Matrix-Ausgangsmaterial der Hydroborierung mit Diboran oder vorzugsweise mit mono- und insbesondere disubstituierten Boranen der allgemeinen Formel $R_1 R_2 BH$ unterwirft, wobei in dieser Formel RI ein Kohlenwasserstoffrest und $R_2$ Wasserstoff oder ebenfalls ein Kohlenwasserstoffrest ist, der mit RI gleich oder von ihm verschieden sein kann oder auch mit RI und dem Bor gemeinsam ein Ringsystem bildet. Solche das Bor substituirenden Kohlenwasserstoffreste weisen jeweils nicht mehr als 15 C-Atome auf. In einer besonders bevorzugten Ausführungsform bilden RI und R2 zusammen mit dem Boratom ein Ringsystem, das die angegebenen Zahlenwerte für die Anzahl der Kohlenstoffatome nicht überschreitet.

Eine besonders geeignete Klasse von Organoborverbindungen für die Herstellung der polymeren Initiatorkomponente sind Organobormonohydridverbindungen, insbesondere Dialkylbormonohydride. Typische Vertreter solcher Borverbindungen sind beispielsweise 9-Borabicyclo[3.3.1]-nonan (9-BBN), Diisopinocampheylboran, Dicyclohexylboran, The-xylboran(2,3-Dimethyl-2butylboran), 3,5-Dimethylborinan und Diisoamylboran. Unter diesen Verbindungen kann das zuerst genannte 9-BBN aus praktischen Gründen bevorzugt sein. Die vorstehend genannten Verbindungen können beispielsweise aus Natriumborhydrid und Bortrifluorid mit geeigneten Olefinen oder Diolefinen hergestellt werden. Auch können zur Darstellung Diboran, dessen Ether-, Aminoder Sulfidkomplexe eingesetzt werden. Eine Zusammenstellung der Herstellungsmöglichkeiten geeigneter Borverbindungen findet sich in der Monographie Herbert C. Brown, 1975 "Organic Synthesis via Boranes", Verlag John Wiley & Sons.

Entscheidende Bedeutung kommt der erfindungsgemäß als Matrix dienenden Esterbasis zu. Das Ausgangsmaterial für diese Matrix kennzeichnet sich dadurch, daß Fettsäuren und/oder Fettalkohle zu den eingangs definierten Estern beziehungsweise Estergemischen umgesetzt sind, die wenigstens in einem Anteil ihrer Fettsäure- und/oder Fettalkohol-Bestandteile additionsbereite Kohlenstoffdoppelbindungen aufweisen. Der Einfachheit halber werden im folgenden diese Bestandteile als ungesättigte Komponenten bezeichnet.

Der Begriff der Fettsäure beziehungsweise des Fettalkohols erfaßt dabei Monocarbonsäuren und Monoalkohole der genannten Art mit einer Kohlenstoffzahl von 8 bis 32 C-Atomen vorzugsweise von 14 bis 22 C-Atomen. Die ungesättigten Fettsäuren beziehungsweise ungesättigten Fettalkohole können synthetischen oder natürlichen Ursprungs sein. Bevorzugt handelt es sich um ein- oder mehrfach olefinisch ungesättigte Alkenmonocarbonsäuren beziehungsweise Monoalkenole der angegebenen Kohlenstoffzahl. Die Kohlenstoffketten dieser Fettsäuren beziehungsweise Fettalkohole können geradkettig und/oder verzweigt sein.

Die den jeweiligen Ester bildenden Gegenkomponenten können Fettalkohole beziehungsweise Fettsäuren aber auchwie eingangs definiert - mehrwertige Alkohole beziehungsweise mehrwertige Carbonsäuren sein. Es ist möglich, daß additionsbereite Kohlenstoff-Doppelbindungen in nur einem Bestandteil - also nur in der Fettsäure beziehungsweise in dem Fettalkohol - vorliegen, ebenso können aber auch beide Ester-bildenden Komponenten olefinische Doppelbindungen enthalten. Zur Herstellung der erfindungsgemäß eingesetzten Boralkylverbindungen wird wenigstens ein substantieller Anteil - wenigsten 50 % - dieser Doppelbindungen der Hydroborierung unterworfen.

In einer bevorzugten Ausführungsform der Erfindung ist die Estermatrix durch Veresterung einer Fettsäure beziehungsweise eines Fettalkohols mit einer mehrfunktionellen Gegenkomponente (Alkohol beziehungsweise Säure) gebildet. Besonders bevorzugte Matrixmaterialien können Ester ungesättigter Fettsäuren mit mehrwertigen Alkoholen sein. Als mehrwertige Ester bildende Reaktionskomponenten sind entsprechende Verbindungen einer Funktionalität bis zu 6 vorzugsweise mit einer Funktionaltät von 2 bis 4 geeignet. So sind in der bevorzugten Ausführungsform der Erfindung als Matrix für die

borhaltigen Substituenten Fettsäuren des angegebenen C-Zahlbereichs mit mehrwertigen Alkoholen - insbesondere mit zweiwertigen, dreiwertigen oder vierwertigen Alkoholen - verestert.

Die polyfunktionelle Esterkomponente besitzt eine vergleichsweise niedrige Anzahl von Kohlenstoffatomen, die im Bereich von 2 bis 10 vorzugsweise im Bereich von 2 bis 6 C-Atomen liegt. Als polyfunktionelle Alkohole eignen sich dementsprechend besonders die niederen Glykole wie Ethylenglykol, Propylenglykol-1,2, Propylenglykol-1,3, die $C_4$-Glykole mit endständigen und/oder innenständigen Hydroxylgruppen beziehungsweise entsprechende $C_5$- und $C_6$-Verbindungen. Ein besonders bevorzugter Alkohol ist weiterhin das Glycerin oder mehrwertige Alkohole von der Art des Pentaerythrits. Umgekehrt können Fettalkohole mit niederen Polycarbonsäuren insbesondere niederen Dicarbonsäuren oder niederen Tricarbonsäuren verestert sein.

In den Rahmen der Erfindung fällt die Verwendung synthetischer oder natürlicher Fette und/oder Öle als Estermatrix für die anschließende Hydroborierung. Grundsätzlich können die ungesättigten Ester in Abmischung mit gesättigten Komponenten, beispielsweise in Abmischung mit gesättigten Estern und/oder als Mischung verschiedenartiger ungesättigter Ester vorliegen.

Die der Hydroborierung zugängliche ethylenische Doppelbindungen aufweisenden Ester beziehungsweise Fette und/ oder Öle können je nach Struktur und Molekulargewicht niedrig-viskos fließfähig bis fest sein. Für einige Anwendungszwecke - beispielsweise auf dem Gebiet der Reaktionsklebstoffe - kann es wünschenswert sein, daß die Organoborborverbindungen bei Raumtemperatur wenigstens viskos fließfähig beziehungsweise streichfähig sind. Für die Wirksamkeit der erfindungsgemäß eingesetzten Organoborverbindungen als Initiatoren ist das allerdings keine Voraussetzung. Im Gegenteil kann die Lagerstabilität von bei Raumtemperatur festen entsprechenden Organoborverbindungen besonders gut sein.

Die ungesättigten Ester beziehungsweise Estergemische wie Fette und Öle besitzen vor der Hydroborierung bevorzugt eine Jodzahl von etwa 5 bis 130.

Besonders geeignet können beispielsweise die folgenden Fette beziehungsweise Öle natürlichen Ursprungs sein:

| Name | Jodzahl | | |
|------|------|------|------|
| Kokosöl | 7,5 | - | 10,5 |
| Palmkernöl | 14 | - | 23 |
| Rindertalg | 40 | - | 48 |
| Palmöl | 44 | - | 54 |
| Schmalz | 55 | - | 57 |
| Spermöl | | 70 | |
| Rizinusöl | 81 | - | 91 |
| Erdnußöl | 84 | - | 100 |
| Rüböl | 97 | - | 108 |

| Name | Jodzahl | |
|---|---|---|
| Baumwollsaatöl | 99 | - 103 |
| Sojabohnenöl | 120 | - 141 |
| Heringsöl | 123 | - 142 |
| Sonnenblumenöl | 126 | - 136 |
| Leinöl | 155 | - 205 |

Ester beziehungsweise Estergemische unter Mitverwendung der folgenden Fettsäuren sind bevorzugte Ausgangsmaterialien für die Estermatrix im Sinne der Erfindung:

| Bezeichnung | Trivialname | Doppelbindung |
|---|---|---|
| Dodecensäure | Lauroleinsäure | 1 |
| Tetradecensäure | Myristoleinsäure | 1 |
| Hexadecensäure | Palmitoleinsäure | 1 |
| Octadecensäure | Ölsäure | 1 |
| Eicosensäure | Gadoleinsäure | 1 |
| Docosensäure | Erucasäure | 1 |
| 12-Oxy-octadecensäure | Ricinolsäure | 1 |
| Octadecadiensäure | Linolsäure | 2 |
| Octadecatriensäure | Linolensäure | 3 |
| Eicosatetraensäure | Arachidonsäure | 4 |
| Docosapentaensäure | Clupanodonsäure | 5 |

Als Edukte für die Hydroborierung ebenfalls geeignet sind Ester von ungesättigten Fettalkoholen mit gesättigten Dicarbonsäuren zum Beispiel der Glykolsäurediester von Oleylalkohol beziehungsweise der Adipinsäureester von Oleylalkohol und vergleichbare Verbindungen.

Zu den bevorzugten Ausführungsformen der Erfindung sind wenigstens etwa 70 % der in der Estermatrix ursprünglich vorhandenen ethylenischen Doppelbindungen hydroboriert. Besonders geeignet sind solche Organoborverbindungen, in denen - bezogen auf den Reaktionsansatz - wenigstens 80 %, vorzugsweise wenigstens 90 % oder sogar wenigstens 95 % der ethylenischen Doppelbindungen der Umsetzung mit den borhaltigen Komponenten zugeführt worden sind.

Zur Hydroborierung werden die ungesättigten Ester unter vollkommenem Sauerstoffausschluß durch Reaktion mit den ausgewählten Borhydridverbindungen umgesetzt. Zweckmäßig wird dabei in Lösungsmitteln gearbeitet. Geeignet sind die bekannten Lösungsmittel für Organoborverbindungen, insbesondere Tetrahydrofuran, Glykol oder Polyether wie Diethylenglykol-dimethylether, aber auch Ester und Halogenkohlenwasserstoffe. Die Umsetzung erfolgt zweckmäßigerweise im Temperaturbereich von etwa 0 bis 100 °C insbesondere im Bereich von etwa Raumtemperatur bis 70 °C.

Die hydroborierten Esterprodukte können dann durch Abziehen des Lösungsmittels isoliert werden. Sie sind je nach Zusammensetzung und Molekulargewicht viskos bis fest. Ihre Lagerung erfolgt zweckmäßigerweise im verschlossenen Gefäß, bevorzugt unter Inertgas, beispielsweise Stickstoff. In Substanz sind diese Boralkylverbindungen gegenüber Luft relativ stabil. Ausgewählte Komponenten können beispielweise einen Tag lang an der Luft in einer offenen Schale gelagert werden und zeigen gleichwohl noch immer Startereigenschaften für die Aushärtung olefinischer Komponenten, die mit der frisch hergestellten oder unter Sauerstoffausschluß gelagerten entsprechenden borhaltigen Verbindung praktisch vergleichbar sind.

Beim Einsatz der neuen Boralkylverbindungen als Starter für Reaktivklebstoffe setzt man von den Borinitiatoren etwa 0,1 bis 40 Gewichtsprozent insbesondere etwa 0,1 bis 30 Gewichtsprozent - jeweils bezogen auf den zu polymerisierenden Anteil ein. Es kann besonders zweckmäßig sein, den erfindungsgemäßen Härter in Mengen von etwa 0,5 - 10 Gewichtsprozent - bezogen auf den zu polymerisierenden Anteil - zu verwenden. Als polymerisierbare Bestandteile kommen die zahlreichen bekannten Verbindungen mit polymerisierbarer ethylenischer Doppelbindung in Betracht wie sie auf den verschiedenen Gebieten der Verklebung von Metall, Holz, Glas, Keramik und/oder Kunststoffen beschrieben sind. Aber auch für den Einsatz auf Sondergebieten beispielsweise auf dem Gebiet chirurgischer Bindemittel zum Verbinden von hartem Gewebe insbesondere Knochen, gewünschtenfalls mit Metallen oder Kunststoffen, oder auf dem Gebiet der dentalmedizinischen Binde- und Füllmaterialien können die neuen Boralkyl-Ester-Verbindungen geeignet sein.

Zur Reaktivitäts-Strukturbeziehung lassen sich die folgenden allgemeinen Aussagen machen:

1. Die Hydroborierungsprodukte von ungesättigten Estern, beispielsweise Ölen oder Fetten beziehungsweise entsprechenden Gemischen, mit Alkylboranen - etwa von der Art des 9-BBN - sind als Polymerisationsinitiatoren aktiver als die entsprechenden Umsetzungsprodukte mit Borwasserstoff. Die zuerst genannten Boralkyl-Ester-Derivate sind zudem wesentlich unempfindlicher gegenüber Luftsauerstoff.

2. Um gleichzeitig hohe Reaktivität und optimale Unempfindlichkeit gegenüber Luftsauerstoff zu erreichen, ist eine möglichst vollständige Hydroborierung der Olefingruppen tragenden Matrix wünschenswert.

3. Hydroborierungsprodukte von Fettsäureestern, bei denen mehrfunktionelle Reaktionskomponenten mitverwendet worden sind, zeichnen sich durch erhöhte Stabilität an Luft aus. So sind die 9-BBN-Hydroborierungsprodukte von Fettsäureestern monofunktioneller Alkohole - beispielsweise niederer Alkanole - an Luft wesentlich instabiler als die vergleichbaren Derivate, die unter Mitverwendung von Glycerin oder Pentaerythrit als Alkoholkomponente hergestellt worden sind.

4. Die Hydroborierungsprodukte von Fetten hoher Jodzahl sind aktiver als die entsprechenden Derivate von Produkten niedriger Jodzahl.

**Beispiele:**

a) Herstellung von 9-BBN-Derivaten von Fetten, Ölen oder Fettsäurederivaten

Zur Befreiung von Restsauerstoff werden die Fette, Öle oder Fettsäurederivate in der gleichen Menge Tetrahydrofuran (THF) gelöst. Im Anschluß wird das Lösungsmittel im Vakuum bei $1,3 \cdot 10^{-4}$ mbar abgezogen. In einer Glovebox werden erneut gleiche Gewichtsteile frischdestilliertes, entgastes THF zugegeben und die Fette, Öle oder Fettsäurederivate gelöst. Unter vollständigem Sauerstoffausschluß werden die in der Tabelle 1 aufgelisteten Mengen an 9-Borabicyclo-[3.3.1]-nonan (9-BBN) zugegeben und das Gemisch so lange gerührt bis das 9- BBN quantitativ in Lösung gegangen ist. Am Anschluß erhitzt man 1 Stunde unter Rühren auf 60 °C. Das THF wird im Vakuum abgezogen und das Vorratsgefäß verschlossen. Die Entnahme von Proben erfolgt unter Schutzgas und völligem Sauerstoffausschluß.

**Tabelle 1**

Umsetzung von Ölen, Fetten und Fettsäurederivaten mit 9-BBN

| Beispiel Nr. | Öl, Fett, Fettsäure- derivat | Jod- zahl | eingesetzte Menge 9-BBN/g pro 100 g Substanz | Modifizierungsgrad an der Doppelbindung % | Produkteigenschaften |
|---|---|---|---|---|---|
| 1 | Palmöl, fest | 51,9 | 24,9 | 100 | viskos, orangefarben |
| 2 | Palmöl, flüssig | 64,4 | 30,5 | 100 | homogen, viskos, orangefarben |
| 3 | Erdnußöl | 92 | 43 | 100 | homogen, viskos, gelb |
| 4 | Leinöl | 178 | 86 | 100 | homogen, viskos, hellgelb |
| 5 | 1,2-Propylenglykol dioleat | 93 | 43,5 | 100 | homogen, niedrigviskos, hellgelb |
| 6 | Pentaerythrittetraoleat | 93 | 43,5 | 100 | homogen, niedrigviskos, hellgelb |
| 7 | Jojobaoel (synthetisch) | 89 | 41,7 | 100 | homogen, niedrigviskos, dunkelgelb |

Der Umsatz der Reaktion wurde anhand des $^1$H-NMR-Spektrums (-CH=CH-Resonanz bei δ = 5,3 ppm) und des $^{13}$C-NMR-Spektrums (-CH=CH-Resonanzen bei δ = 129,5 und 129,8 ppm) verfolgt. Der Umsatz an der Doppelbindung beträgt im Beispiel 3 70 ± 5 %.

b) Herstellung von BH3-Derivaten von Fetten, Ölen oder Fettsäurederivaten

Zur Befreiung von Restsauerstoff wurden die Fette, Öle oder Fettsäurederivate in der gleichen Menge THF gelöst. Im Anschluß wird das Lösungsmittel im Vakuum bei 1,3. 10$^{-4}$ mbar abgezogen. In das Fettderivat werden im Stickstoffstrom bei konstant 5 °C 0,33 mol Diboran/ THF pro Doppelbindung zugetropft. Die Reaktion ist exotherm. Das Reaktionsgemisch wird auf Raumtemperatur erwärmt und im Anschluß eine weitere Stunde bei 60 °C gerührt. Die THF-Lösung wird unter Stickstoff filtriert, das THF im Hochvakuum abgezogen und das Vorratsgefäß verschlossen. Die Entnahme von Proben erfolgt unter Schutzgas und völligem Sauerstoffaus-schluß. Die Beispiele sind in der Tabelle 2 zusammengefaßt.

**Tabelle 2**

Umsetzung von Ölen, Fetten und Fettsäurederivaten mit BH3

| Nr. | Öl, Fett, Fettsäure-derivat | Jod-zahl | Modifizierungsgrad an der Doppelbindung | Produkteigenschaften |
|-----|------|------|------|------|
| 8 | Erdnußöl | 92 | 100 | viskos, klar |

c) Verwendung der dargestellten Boralkyle auf Fettbasis als Härter für Monomerklebstoffe

**Allgemeine Vorschrift**

In einem Becherglas wurden 40 g Polymethacrylsäuremethylester (PMMA, handelsübliches Pulver)+) in 45 g Methacrylsäuremethylester (MMA) und 5 g Methacrylsäure (MAS) unter Rühren gelöst. Zu jeweils 5 g dieser Mischung wurden unter weiterem intensiverem Rühren zwischen 1,5 und 23 Gewichtsprozent der bereits unter a) und b) beschriebenen Boralkylstarter (siehe Tabellen 1 und 2) zugegeben. Die Topfzeiten der Mischungen variieren zwischen 1 und 15 Minuten. Mit diesen Klebstoffen wurden innerhalb der Topfzeit sandgestrahlte und entfettete Eisenbleche verklebt und nach 24 Stunden die Festigkeiten im Zugscherversuch nach DIN 53 281 / 3 gemessen. Die Ergebnisse sind in der Tabelle 3 zusammengestellt.

Zum Nachweis der hohen Stabilität der dargestellten Boralkyle auf Fettsäurebasis gegenüber Luftsauerstoff wurden sie in einer weiteren Versuchsreihe im offenen Gefäß zwischen 24 und 72 Stunden an der Luft gelagert und im Anschluß als Härter eingesetzt und getestet. Die Topfzeiten und Zugscherfestigkeiten sind in der Tabelle 3 in Klammern gesetzt.

+)Plexigum MB 319 der Firma RÖHM, Darmstadt

**Tabelle 3**

Topfzeiten und Zugscherfestigkeiten auf sandgestrahlten und entfetteten Eisenblechprüfkörpern bei der Härtung von Methacrylatklebstoffen (40 g PMMA, 45 g MMA, 5 g MAS) mit den Boralkylen aus den Tabellen 1 und 2. Bei den in Klammern gesetzten Meßergebnissen wurde der Boralkylhärter vor der Verwendung 24 Stunden bei Raumtemperatur an der Luft gelagert.

Boralkyl aus Beispiel 1

| Härterkonzentration Gew.-% | Topfzeit min. | Zugscherfestigkeiten Nmm$^{-2}$ |
|---|---|---|
| 1,5 | 11 | 3 |
| 3 | 9 | 4 |
| 5 | 8 | 6 |
| 10 | 5 | 8 |
| 23 | 3 | 3 |

Nach 24stündiger Lagerung an der Luft ist dieses Boralkyl nicht mehr aktiv.

**Tabelle 3** (Fortsetzung)

Boralkyl aus Beispiel 2

| Härterkonzentration Gew.-% | Topfzeit min. | Zugscherfestigkeiten $Nmm^{-2}$ |
|---|---|---|
| 1,5 | 10　(14) | 14　(0) |
| 3 | 7,5 (10) | 21　(7) |
| 5 | 6,5 ( 6,5) | 20　(14) |
| 10 | 5　( 6) | 16　(14) |
| 23 | 2　( 2) | 10　(13) |

Bei den in Klammern gesetzten Meßergebnissen wurde der Boralkylhärter vor der Verwendung 24 Stunden bei Raumtemperatur an der Luft gelagert.

Boralkyl aus Beispiel 3

| Härterkonzentration Gew.-% | Topfzeit min. | Zugscherfestigkeiten $Nmm^{-2}$ |
|---|---|---|
| 1,5 | 6 | 23 |
| 3 | 4,5 (6,5) | 25　(20) |
| 5 | 3,5 (5) | 22　(20) |
| 10 | 3　(3) | 17　(14) |
| 23 | 1,5 (1) | 17　(11) |

Bei den in Klammern gesetzten Meßergebnissen wurde der Boralkylhärter vor der Verwendung 48 Stunden bei Raumtemperatur an der Luft gelagert.

**Tabelle 3** (Fortsetzung)

Boralkyl aus Beispiel 4

| Härterkonzentration Gew.-% | Topfzeit min. | | Zugscherfestigkeiten Nmm$^{-2}$ | |
|---|---|---|---|---|
| 1,5 | 3 | (4) | 28 | (28) |
| 3 | 3 | (3) | 28 | (28) |
| 5 | 2,5 | (2,5) | 23 | (24) |
| 10 | 1,5 | (2,5) | 15 | (18) |
| 23 | 1 | (1) | 14 | (11) |

Bei den in Klammern gesetzten Meßergebnissen wurde der Boralkylhärter vor der
Verwendung 24 Stunden bei Raumtemperatur an der Luft gelagert.

Boralkyl aus Beispiel 5

| Härterkonzentration Gew.-% | Topfzeit min. | | Zugscherfestigkeiten Nmm$^{-2}$ | |
|---|---|---|---|---|
| 1,5 | 6 | (8) | 26 | (10) |
| 3 | 5,5 | (5,5) | 26 | (21) |
| 5 | 5 | (5) | 25 | (17) |
| 10 | 3 | (3) | 20 | (18) |
| 23 | 1 | (2) | 14 | (11) |

Bei den in Klammern gesetzten Werten wurde der Boralkylhärter vor der
Verwendung 72 Stunden bei Raumtemperatur an der Luft gelagert.

**Tabelle 3** (Fortsetzung)

Boralkyl aus Beispiel 6

| Härterkonzentration Gew.-% | Topfzeit min. | Zugscherfestigkeiten Nmm$^{-2}$ |
|---|---|---|
| 1,5 | 5 (9) | 26 (17) |
| 3 | 5 (7,5) | 26 (20) |
| 5 | 4 (5,5) | 23 (19) |
| 10 | 3,5 (4) | 19 (15) |
| 23 | 0,5 (2) | 13 (11) |

Bei den in Klammern gesetzten Werten wurde der Boralkylhärter vor der Verwendung 72 Stunden bei Raumtemperatur an der Luft gelagert.

Boralkyl aus Beispiel 7

| Härterkonzentration Gew.-% | Topfzeit min. | Zugscherfestigkeiten Nmm$^{-2}$ |
|---|---|---|
| 1,5 | 6,5 (7,5) | 20 (16) |
| 3 | 5,5 (6,5) | 26 (20) |
| 5 | 5 (5,5) | 24 (17) |
| 10 | 2,5 (3,5) | 21 (16) |
| 23 | 1 (2) | 18 (11) |

Bei den in Klammern gesetzten Werten wurde der Boralkylhärter vor der Verwendung 72 Stunden bei Raumtemperatur an der Luft gelagert.

**Tabelle 3** (Fortsetzung)

Boralkyl aus Beispiel 8

| Härterkonzentration Gew.-% | Topfzeit min. | Zugscherfestigkeiten $Nmm^{-2}$ |
|---|---|---|
| 1,5 | 15 | 7 |
| 3 | 6 | 6 |
| 5 | 2 | 9 |
| 10 | 1,5 | 6 |
| 23 | 1 | 1 |

Nach 24 stündiger Lagerung an der Luft ist dieses Boralkyl nicht mehr aktiv.

## Beispiele für Anwendung in der Medizin

### Probekörper

Knochenprüfkörper aus Rindercortalis mit den Abmessungen 50 x 20 x 5 mm wurden in der Mitte - exakt rechtwinklig zu den Seitenflächen (50 x 5 mm) - mit einer Diamant-Trennsäge unter Wasserspülung osteotomiert.

Auf diese Weise wurden paßgenaue Fügeteile mit einer Berührungsfläche von 1 cm² hergestellt.

### Probekörpervorbehandlung

Die nicht weiter vorbehandelten Knochenprüfkörper wurden 2 Stunden in eine 37 °C warme Ringerlösung gelegt, um die Knochen intensiv zu benetzen.

### Verklebung

2-K-Klebstoffe
Klebstoffharz (A) und Härter (B) werden gemischt. Das Gemisch wird auf den feuchten Knochen aufgetragen.

Die Prüfkörper werden gefügt, in eine Presse aus PVC überführt und mit 5 N belastet. Die Klebepresse verhindert ein seitliches Verschieben der Prüfkörper während der Aushärtung.

### Aushärtebedingungen

Die gefügten Knochen werden in der Klebpresse 20 min lang in einer Klimakammer bei 37 °C und ca. 100 % relativer Luftfeuchtigkeit gelagert. Im Anschluß werden sie der Klebepresse entnommen und 20 Std. in Ringerlösung bei 37 °C gelagert.

### Festigkeitsmessung

Die Zugfestigkeit wird an einer Universalprüfmaschine mit einer Vorschubgeschwindigkeit von 1,0 mm/min zerrissen.

Aufgrund der durch das natürliche Substrat vorgegebenen breiten Streuung der Messwerte wurden jeweils die Festigkeitsbereiche aus 6 Einzelversuchen angegeben.

### Harzkomponenten A

| A 1 | Methylmethacrylat | 50 Gew.% |
| | IPA 514[1] | 5 Gew.% |
| | Polymethylmethacrylat[2] | 45 Gew.% |

| A 2 | Methylmethacrylat | 50 Gew.% |
| | IPA 514 | 5 Gew.% |
| | Perfluoralkylmethacrylat[3] | 5 Gew.% |
| | Polymethylmethacrylat | 40 Gew.% |

1) Gemisch aus Methacryloyloxyethylphosphat und Bismethacryloyloxyethylphosphat der Johucu Chemical, Japan.
2) Plexigum MB 319 der Firma Röhm, Darmstadt.
3) Umsetzungsprodukt von Perfluordodecansäure mit Glycidylmethacrylat.

Zur Herstellung der Harzkomponenten wird Polymethylmethacrylat unter intensivem Rühren in den entsprechenden Monomeren gelöst.

### Initiatorkomponenten B

Zur Befreiung von Restsauerstoff wird Leinöl gemäß Tabelle 1 in der gleichen Menge THF gelöst. Im Anschluß wird das Lösungsmittel im Vakuum bei 1,3. 10⁻⁴ mbar abgezogen. In einer Glovebox werden erneut gleiche Gewichtsteile frischdestilliertes, entgastes THF zugegeben und das Öl gelöst. Unter vollständigem Sauerstoffausschluß wird die in der Tabelle 1 aufgelistete Menge an 9-Borabicyclo [3.3.1]-nonan (9-BBN) zugegeben und das Gemisch so lange gerührt, bis das 9-BBN quantitativ in Lösung gegangen ist. Im Anschluß erhitzt man 1 Stunde unter Rühren auf 60 °C. Das THF wird im Vakuum abgezogen und das Vorrats-gefäß verschlossen. Die Entnahme von Proben erfolgt unter Schutzgas und vollständigem Sauerstoffausschluß.

### Tabelle 4

Darstellung der Initiatorkomponente B

| Nr. | Öl | Jod-zahl | eingesetzte Menge 9-BBN/g pro 100 g Substanz | Modifizierungsgrad an der Doppelbindung | Produkteigenschaften |
|---|---|---|---|---|---|
| B | Leinöl | 178 | 86 | 100 | homogen, viskos, hellgelb |

## Ergebnisse

Die gemessenen Zugfestigkeiten sind in der Tabelle 5 zusammenfefaßt.

**Tabelle 5**

Übersicht über die gemessenen Zugfestigkeiten (N cm$^{-2}$

| Harzkomponente A | Härterkomponente B |
|---|---|
| A 1 | 200 – 600 |
| A 2 | 250 – 780 |

## Patentansprüche

1. Verfahren zum Härten von Reaktivklebstoffen, welche auf Basis von polymerisierbaren Monomeren aufgebaut sind, durch radikalische Polymerisation mit Boralkylverbindungen als Initiatoren, dadurch gekennzeichnet, daß als Boralkylverbindungen Hydroborierungsprodukte eingesetzt werden aus

a) Borwasserstoff und/oder wenigstens eine B-H-Bindung; aufweisenden Organoborverbindungen, deren Kohlenwasserstoffreste jeweils bis zu 15 C-Atome aufweisen und

b) Estern oder Estergemischen von Fettsäuren und/oder Fettalkoholen mit jeweils 8 - 32 C-Atomen, die wenigstens in einem Anteil ihrer Fettsäure- und/oder Fettalkoholreste wenigstens eine additionsbereite C=C-Doppelbindungen bei einer Ausgangsjodzahl der Ester von 1 - 205 aufweisen, und ihrerseits Umsetzungsprodukte solcher Fettsäuren mit den Fettalkoholen oder Ester solcher Fettsäuren mit mehrwertigen Alkoholen oder. Ester solcher Fettalkohole mit mehrwertigen Carbonsäuren sind, wobei die mehrwertigen Alkohole bzw. mehrwertigen Carbonsäuren jeweils 2 - 10 C-Atome bei einer Funktionalität bis 6 aufweisen und wobei weiterhin

c) die im Ausgangsester vorliegenden C=C-Doppelbindungen zu wenigstens 50 % der Hydroborierung unterworfen worden sind.

2. Verfahren nach Anspruch 1, dadurch gekennzeichnet, daß Boralkylverbindungen eingesetzt werden, deren mehrwertige Ester-bildenden Komponenten eine Funktionalität von 2 - 4 aufweisen und bevorzugt 2 - 6 C-Atome enthalten.

3. Verfahren nach den Ansprüchen 1 und 2, dadurch gekennzeichnet, daß Boralkylverbindungen eingesetzt werden, in denen die Fettsäure- bzw. Fettalkoholreste 14 - 22 C-Atome enthalten.

4. Verfahren nach den Ansprüchen 1 bis 3, dadurch gekennzeichnet, daß als Boralkylverbindungen Hydroborierungsprodukte ungesättigter Fette und/oder Öle, insbesondere natürlichen Ursprungs, eingesetzt werden, die insbesondere auch gesättigte Ester in Abmischung enthalten können.

5. Verfahren nach den Ansprüchen 1 bis 4, dadurch gekennzeichnet, daß die Boralkylverbindungen Hydroborierungsprodukte von Estern mit einer Ausgangsjodzahl im Bereich 5 bis 130 sind.

6. Verfahren nach den Ansprüchen 1 bis 5, dadurch gekennzeichnet, daß Boralkylverbindungen eingesetzt werden, in denen wenigstens 70 % der ethylenischen Doppelbindungen des Esters oder Estergemisches hydroboriert worden sind.

7. Verfahren nach den Ansprüchen 1 bis 6, dadurch gekennzeichnet, daß die hydroborierten Ester Organoborreste mit Alkyl-, Cycloalkyl- und/oder Arylgruppen enthalten und auch noch eine BH-Bindung aufweisen können.

8. Verfahren nach den Ansprüchen 1 bis 7, dadurch gekennzeichnet, daß man Reaktionsklebstoffe beim Verbinden von Metall, Holz, Glas, Keramik oder Kunststoffen härtet.

9. Boralkylverbindungen die hergestellt worden sind durch Umsetzung von
a) Borwasserstoff und/oder wenigstens eine B-H-Bindung aufweisenden Organoborverbindungen mit jeweils bis zu 15 C-Atomen in den Kohlenwasserstoffresten und
b) Estern oder Estergemischen von Fettsäuren und/oder Fettalkoholen mit jeweils 8 - 32 C-Atomen, die wenigstens in einem Anteil Ihrer Fettsäure- oder Fettalkoholreste wenigstens eine additionsbereite C=C-Doppelbindung bei einer Ausgangsjodzahl der Ester von 1 - 205 aufweisen, dadurch gekennzeichnet,
daß sie als Reaktionskomponente b Ester von Fettsäuren mit mehrwertigen Alkoholen bzw. Ester von Fettalkoholen mit mehrwertigen Carbonsäuren enthalten, wobei diese mehrwertigen Komponenten bei einer Funktionalität bis 6, vorzugsweise von 2 - 4, jeweils 2 - 10 C-Atome, vorzugsweise 2 - 6 C-Atome aufweisen und wobei die in den Estern ursprünglich vorliegende(n) C=C-Doppelbindung(en) zu wenigstens 50 %, vorzugsweise zu wenigstens 70 %, hydroboriert sind.

10. Verwendung der Boralkylverbindungen nach Anspruch 9 als Härterkomponente für Reaktionsklebstoffe, die auch als chirurgische Bindemittel zum Verbinden von Hartgewebe, insbesonde Knochen, oder als dentalmedizinisches Binde- und Füllmaterial geeignet sind.

## Claims

1. A process for curing reactive adhesives based on polymerizable monomers by radical polymerization with boronalkyl compounds as initiators, characterized in that the boronalkyl compounds used are hydroboration products of
a) boron hydride and/or organoboron compounds containing at least one B-H-bond of which the hydrocarbon radicals contain up to 15 C-atoms and
b) esters or ester mixtures of fatty acids and/or fatty alcohols containing from 8 to 32 C-atoms which, in at least part of their fatty acid and/or fatty alcohol residues, contain at least one C=C-double bond capable of addition for a starting iodine number of the esters of from 1 to 205 and which in turn are reaction products of such fattyacids with the fatty alcohols or esters of such fatty acids with polyhydric alcohols or esters of such fatty alcohols with polybasic carboxylic acids, the polyhydric alcohols and polybasic carboxylic acids containing from 2 to 10 C-atoms for a functionality of up to 6 and
c) at least 50% of the C=C-double bonds present in the starting ester having been subjected to hydroboration.

2. A process as claimed in Claim 1, characterized in that boronalkyl compounds of which the polyfunctional ester-forming components have a functionality of from 2 to 4 and preferably contain from 2 to 6 C-atoms are used.

3. A process as claimed in Claims 1 and 2, characterized in that boronalkyl compounds in which the fatty acid or fatty alcohol residues contain from 14 to 22 C-atoms are used.

4. A process as claimed in Claims 1 to 3, characterized in that the boronalkyl compounds used are hydroboration products of unsaturated fats and/or oils, particularly of natural origin, which in particular may even contain unsaturated esters in admixture.

5. A process as claimed in Claims 1 to 4, characterized in that the boronalkyl compounds are hydroboration products of esters having a starting iodine number of from 5 to 130.

6. A process as claimed in Claims 1 to 5, characterized in that boronalkyl compounds in which at least 70% of the ethylenic double bonds of the ester or ester mixture have been subjected to hydroboration are used.

7. A process as claimed in Claims 1 to 6, characterized in that the hydroborated esters contain organoboron residues containing alkyl, cycloalkyl and/or aryl groups and may also contain a BH-bond.

8. A process as claimed in Claims 1 to 7, characterized in that reactive adhesives are cured in the bonding of metals, wood, glass, ceramics or plastics.

9. Boronalkyl compounds which have been produced by reaction of

a) boron hydride and/or organoboron compounds containing at least one B-H-bond of which the hydrocarbon radicals contain up to 15 C-atoms and

b) esters or ester mixtures of fatty acids and/or fatty alcohols containing from 8 to 32 C-atoms which, in at least part of their fatty acid or fatty alcohol residues, contain at least one C=C-double bond capable of addition for a starting number of the esters of from 1 to 205, characterized in that they contain as reaction component b) esters of fatty acids with polyhydric alcohols or esters of fatty alcohols with polybasic carboxylic acids, these polyfunctional components containing from 2 to 10 and preferably from 2 to 6 C-atoms for a functionality of up to 6 and preferably from 2 to 4 and at least 50% and preferably at least 70% of the C=C-double bonds originally present in the esters having been subjected to hydroboration.

10. The use of the boronalkyl compounds claimed in Claim 9 as curing component for reactive adhesives which are also suitable as surgical adhesives for bonding hard tissue, particularly bones, or as a dental binder and filler.


**Revendications**

1- Procédé pour faire durcir des adhésifs réactifs préparés à base de monomères polymérisables, par polymérisation radicale, avec des composés du bore comme initiateurs, caractérisé en ce que, comme composés alcoylés du bore, on se sert de:

a) borane et/ou, au moins un composé organique du bore comportant au moins une liaison B-H, dont les restes hydrocarbures comportent toujours jusqu'à 15 atomes de carbone, et,

b) des esters ou des mélanges d'esters d'acides et/ou d'alcools gras, avec chaque fois 8 à 32 atomes de C, comportant, au moins une partie constituante de leurs restes acides gras ou alcools gras, au moins une double liaison C=C, capable d'une addition, avec au départ, un indice d'iode de l'ester de 1 à 205, et sont, pour leur part des produits de la réaction d'acides gras de ce genre avec des alcools gras ou des esters de ces acides gras avec des alcools gras ou des esters de ces acides gras avec des alcools polyvalents ou des esters de ces acides gras avec des acides carboxyliques polyvalents, les alcools polyvalents, ou les acides carboxyliques polyvalents présentant chaque fois 2 à 10 atomes C, avec une fonctionnalité pouvant aller jusque 6 et, en outre,

c) les doubles liaisons C=C présentes dans l'ester de départ ont été soumises, pour au moins 50%, à un traitement hydroborique.

2- Procédé suivant la revendication 1, caractérisé en ce que l'on utilise des composés alcoylés du bore dont les composants, formant les esters polyvalents, présentent une fonctionnalité de 2 à 4, et contiennent, de préférence, de 2 à 6 atomes de C.

3- Procédé suivant les revendications 1 et 2, caractérisé en ce que sont mis en oeuvre des composés alcoylés du bore où les restes d'acides gras ou d'alcools gras contiennent de 18 à 22 atomes de C.

4- Procédé suivant les revendications 1 à 3, caractérisé en ce que, comme composés alcoylés du bore, on utilise des graisses et/ou des huiles insaturées en particulier d'origine naturelle, qui ont subi un traitement hydroborique, qui peuvent aussi, en particulier, contenir des esters saturés qui y seraient mélangés.

5- Procédé suivant les revendcations 1 à 4 caractérisé en ce que, les composés alcoylés du bore sont des produits d'un traitement hydroborique d'esters présentant au départ un indice d'iode de l'ordre de 5 à 130.

6- Procédé suivant les revendications 1 à 5, caractérisé en ce que l'on utilise des composés alcoylés du bore dans lesquels au moins 70% des doubles liaison éthyléniques de l'ester ou du mélange d'esters ont subi une réaction hydroborique.

7- Procédé suivant les revendications 1 à 6, caractérisé en ce que les esters hydroboriques contiennent des restes organoboriques avec des groupes alcoyl-, cycloalcoyl et/ou aryl, et peuvent présenter aussi, de plus, une liaison B-H.

8- Procédé suivant les revendications 1 à 7, caractérisé en ce que l'on fait durcir les adhésifs réactifs pour le collage de métaux, bois, verre, céramiques ou matières plastiques.

9- Composés alcoylés du bore qui ont été fabriqués par réaction de:

a) borane et/ou composés organiques du bore, comportant au moins une liaison B-H, avec toujours jusqu'à 15 atomes C dans les restes hydrocarbure et,

b) des esters ou mélanges d'esters d'acides gras et/ou d'alcools gras avec chaque fois 8 à 32 atomes C qui présentent, au moins dans une partie constituante de leurs restes acides gras ou alcools gras, au moins une double liaison C=C, capable de former une addition, avec, au départ, un indice d'iode de l'ester de 1 à 205, caractérisé en ce qu'ils contiennent, comme composants de la réaction b des esters d'acides gras avec des alcools polyvalents, ou des esters d'alcools gras avec des acides carboxyliques polyvalents, ces composants polyvalents comportant, avec une fonctionnalité pouvant aller jusqu'à 6, de préférence de 2 à 4, chaque fois 2 à 10 atomes C, de préférence 2 à 6 atomes C, et la ou les doubles liaisons, présentes primitivement dans les esters, ont subi, pour au moins 50%, ou mieux pour au moins 70%, une réaction hydroborique.

10- Utilisation des composés alcoylés du bore suivant la revendication 9 comme composants durcisseurs pour des adliésifs réactifs, qui peuvent aussi convenir comme liants pour des opérations chirurgicales pour lier des tissus durs, en particulier des os, ou, en médecine dentaire, comme liants et matière de remplissage.